# EUROPEAN PATENT APPLICATION

(11) **EP 4 628 501 A1**
(43) Date of publication of application: **08.10.2025**
(21) Application number: 23898219.3
(22) Date of filing: 24.11.2023
(51) Int. Cl.: C07K 14/47, C07K 7/06, A61K 47/64, A61K 31/704, A61P 3/04, A61P 29/00

(54) **CONJUGATE COMPRISING PEPTIDE OF NOVEL SEQUENCE AND GLYCYRRHIZIN, AND PHARMACEUTICAL COMPOSITION FOR PREVENTING OR TREATING OBESITY COMPRISING SAME**

(30) Priority: 30.11.2022 KR 20220164307; 06.02.2023 KR 20230015492
(71) Applicant: IUCF-HYU (Industry-University Cooperation Foundation Hanyang University), Seoul 04763 (KR)
(72) Inventor: LEE, Dong Yun, Seoul 04763 (KR); MIN, Jae Hong, Seoul 04763 (KR); PRISCILLA, Lia, Seoul 04763 (KR)
(74) Representative: dompatent von Kreisler Selting Werner - Partnerschaft von Patent- und Rechtsanwälten mbB
(86) International application number: PCT/KR2023/019124
(87) International publication number: WO 2024/117688

(57) **Abstract**

One aspect of the present invention relates to: a conjugate comprising a peptide of a novel sequence and glycyrrhizin; and a pharmaceutical composition for preventing or treating obesity, the composition comprising the conjugate. The conjugate and composition comprising same according to one aspect were found to inhibit hypertrophy of fat cells, increase the cell membrane expression level of ATP-binding cassette transporter A1 (ABCA1), and reduce the secretion of tumor necrosis factor-α (TNF-α). In addition, the conjugate and composition comprising same were found to remain in a greater amount in the blood and have better pharmacokinetic properties than glycyrrhizin used by itself, and thus can be used in the obesity prevention and/or treatment market/industry.

## Description

### [Technical Field]

The present invention relates to: a conjugate including a peptide having a novel sequence and glycyrrhizin; and a pharmaceutical composition for preventing or treating obesity, the composition including the conjugate.

### [Background Art]

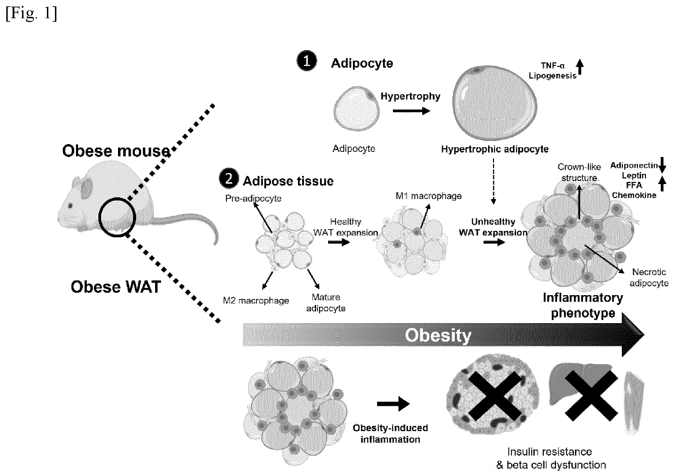

Obesity-induced inflammation is a chronic inflammatory response that occurs from obese adipose tissue and is known to cause serious complications such as diabetes and heart disease. The development of obese adipose tissue is proportional to the progression of obesity, and unlike healthy adipose tissue, the secretion amount of inflammatory factors increases significantly. Since the development of such obese adipose tissue begins with the hypertrophy process of adipocytes due to continuous overnutrition, a mechanism that induces anti-inflammatory actions by inhibiting the adipocyte hypertrophy process is necessary for effective treatment of obesity-induced inflammation (FIG. 1).

Glycyrrhizin (GL) is a low-molecular weight anti-inflammatory drug known to have an anti-inflammatory effect by participating in intracellular cholesterol metabolism. However, since it does not target adipose tissue, it is not suitable for the treatment of obesity-induced inflammation when it is used alone.

Therefore, to solve the above-described problems, the present inventors developed an adipose tissue-targeting glycyrrhizin conjugate (GL-PEG-AHP) using an adipose tissue homing peptide (AHP) targeting prohibitin (PHB), an adipocyte-specific membrane protein.

### Related Art Documents

Korea Patent Publication No. 10-2007276

### [Disclosure]

### [Technical Problem]

One aspect provides a peptide consisting of an amino acid sequence of SEQ ID NO. 1.

Another aspect provides a conjugate including: the peptide consisting of the amino acid sequence of SEQ ID NO. 1; and glycyrrhizin.

Still another aspect provides a pharmaceutical composition for preventing or treating obesity, the pharmaceutical composition including the conjugate.

Yet another aspect provides a health functional food for preventing or ameliorating obesity, the health functional food including the conjugate

Yet another aspect provides a method of preventing or treating obesity, the method including a step of administering the conjugate to an individual in need thereof.

Yet another aspect provides a use of the conjugate for preparing a medicine for preventing or treating obesity.

### [Technical Solution]

One aspect provides a peptide consisting of an amino acid sequence of SEQ ID NO. 1.

In one embodiment, the peptide may include an amino acid sequence of GKGRRAKDC (SEQ ID NO: 1).

In one embodiment, the peptide may include a prohibitin (PHB) target sequence of Annexin A2 (ANXA2).

Another aspect provides a conjugate including: the peptide consisting of the amino acid sequence of SEQ ID NO. 1; and glycyrrhizin.

The "peptide" may be within the above-described range.

The term "glycyrrhizin" is a component extracted from licorice and is known as a factor that regulates glucocorticoids by acting on the 11β-hydroxysteroid dehydrogenase type 1 (11beta-HSD1) hormone. In addition, it lowers insulin resistance, thereby reducing lipolysis that occurs in adipocytes.

In one embodiment, the peptide and the glycyrrhizin may be linked through a cross-linking agent.

In one embodiment, the cross-linking agent may be one or more selected from the group consisting of polyethylene glycol (PEG), 1,4-butanediol diglycidyl ether (BDDE), 1,3-butadiene diepoxide, divinyl sulfone (DVS), glycol chitosan, gelatin methacrylate, poly(lactic-co-glycolic acid) (PLGA), hyaluronic acid, and alginate.

In one embodiment, the cross-linking agent may be PEG.

In one embodiment, the PEG may be thiol-polyethylene-glycol-amine.

In one embodiment, the conjugate may be bonded by a first bond by which the peptide and the cross-linking agent are bonded; and

a second bond by which the glycyrrhizin and the cross-linking agent are bonded.

In one embodiment, the first bond may be a disulfide bond.

In one embodiment, the disulfide bond may formed by a reaction between a thiol group of the peptide and a thiol group of the cross-linking agent.

In one embodiment, the second bond may be an amide bond.

In one embodiment, the amide bond is formed by a reaction between a carboxyl group of the glycyrrhizin and an amine group of the cross-linking agent.

Still another aspect provides a pharmaceutical composition for preventing or treating obesity, the pharmaceutical composition including the conjugate

The "conjugate" may be within the above-described range.

The term "obesity" refers to a state in which excess energy causes an increase in the weight and number of adipocytes in the body, resulting in excessive accumulation of adipose tissue. When the state of obesity persists, abnormalities in the body's metabolic process occur, resulting in metabolic disease or metabolic syndrome, and specifically, one or more symptoms of insulin resistance, type 2 diabetes, hyperlipidemia, fatty liver, or inflammation may appear along with the obesity state.

The term "prevention" may refer to any action that prevents an individual from becoming obese or delays a disease caused by obesity by administering a pharmaceutical composition according to one aspect.

The term "treatment" may refer to any action that ameliorates or beneficially changes symptoms of obesity in an individual by administering a pharmaceutical composition according to one aspect.

The term "administration" refers to introducing a certain substance to an individual in an appropriate manner, and the term "individual" refers to all living organisms, including humans, rats, mice, and livestock, that may have obesity. A specific example may be a mammal, including humans.

In one embodiment, the concentration of the composition may be 10 µM to 500 µM.

In one embodiment, the concentration of the composition may be 10 µM to 500 µM, 10 µM to 460 µM, 10 µM to 430 µM, 15 µM to 500 µM, 15 µM to 460 µM, 15 µM to 430 µM, 20 µM to 500 µM, 20 µM to 460 µM, or 20 µM to 430 µM.

In one embodiment, the composition may inhibit the hypertrophy of an adipocyte.

In one embodiment, to confirm the anti-hypertrophy effect of GL derivatives according to the concentration in adipocytes in which obesity is induced at a cell experiment level, each group (control, GL, PEG, AHP, GL-PEG and GL-PEG-AHP) was treated with various concentrations (25 µM, 50 µM, 100 µM, 200 µM, 400 µM) on day 14 of differentiation, one day before free fatty-acid (FFA) treatment, and then treated with FFA for three days until day 18 of differentiation to induce an obesity model. As a result, it was confirmed that the size of adipocytes was more significantly reduced in the drug pretreatment group (see Example 10).

In one embodiment, the composition may reduce the secretion amount of tumor necrosis factor-α (TNF-α).

In one embodiment, to determine the secretion amount of TNF-α of obese adipocytes, each group (control, GL, PEG, AHP, GL-PEG, and GL-PEG-AHP) was treated for 24 hours one day before treating the obesity adipocyte model with FFA. As a result, it was confirmed that the secretion amount of TNF-α was significantly reduced (see Example 13).

In one embodiment, the composition may increase the cell membrane expression level of ATP-binding cassette 1 transporter (ABCA1).

In one embodiment, the cell membrane expression level of ABCA1 in adipocytes according to GL derivatives was confirmed through fluorescence images, and it was confirmed that the cell membrane expression level of ABCA1 significantly increased in the GL derivative groups (see Example 15).

In one embodiment, the composition may have a blood residual concentration that is 1.5 to 3 times that of the control group.

The term "control group" refers to a group that is left as is without changing the experimental conditions as for the experimental group to contrast the results with the experimental group.

In one embodiment, the control group refers to the group treated with glycyrrhizin.

In one embodiment, the blood residual concentration of the GL derivatives was measured to calculate pharmacokinetic indices. As a result, it was confirmed that the blood residual concentration of GL-PEG-AHP was about 2.3 times that of the GL group (see Example 17).

In addition, the pharmaceutical composition may be provided as a pharmaceutical composition containing the active ingredient alone or containing one or more pharmaceutically acceptable carriers, excipients, or diluents.

Specifically, the carrier may be, for example, a colloidal suspension, powder, a saline solution, a lipid, a liposome, a microsphere, or a spherical nanoparticle. They may be complexed or associated with a carrier vehicle and may be delivered *in vivo* using carrier systems known in the art, such as lipids, liposomes, microparticles, gold, nanoparticles, polymers, condensation agents, polysaccharides, polyamino acids, dendrimers, saponins, adsorption enhancing substances, or fatty acids.

When the pharmaceutical composition is formulated, it may be formulated using commonly used diluents or excipients such as lubricants, sweeteners, flavoring agents, emulsifiers, suspending agents, preservatives, fillers, bulking agents, binders, wetting agents, disintegrants, and surfactants. Solid preparations for oral administration may include tablets, pills, powder, granules, capsules, and the like, and these solid preparations may be prepared by mixing at least one excipient, for example, starch, calcium carbonate, sucrose or lactose, gelatin, and the like, with the composition. In addition to simple excipients, lubricants such as magnesium stearate and talc may also be used. Liquid preparations for oral administration include suspensions, oral solutions, emulsions, syrups, and the like, and in addition to commonly used simple diluents such as water and liquid paraffin, various excipients such as wetting agents, sweeteners, flavoring agents, and preservatives may be included. Preparations for parenteral administration may include sterile aqueous solutions, non-aqueous solvents, suspensions, emulsions, lyophilized preparations, and suppositories. As non-aqueous solvents and suspending agents, propylene glycol, polyethylene glycol, vegetable oils such as olive oil, and injectable esters such as ethyl oleate may be used. As a base for suppositories, Witepsol, Macrogol, Tween 61, cacao oil, laurel oil, glycerol, gelatin, or the like may be used. When manufactured in the form of eye drops, known diluents or excipients may be used.

The pharmaceutical composition may be provided as a mixture with another pharmaceutical composition for preventing or treating obesity, and the other pharmaceutical composition for preventing or treating obesity may be a conventionally known pharmaceutical composition for preventing or treating obesity or a newly developed pharmaceutical composition for preventing or treating obesity.

When the pharmaceutical composition further includes another pharmaceutical composition for preventing or treating obesity or is provided as a mixture with another pharmaceutical composition for preventing or treating obesity, it is important to mix an amount that may achieve a maximum effect with a minimum amount without causing side effects, and this may be easily determined by one of ordinary skill in the art.

The pharmaceutical composition may be administered in parallel with another pharmaceutical composition for preventing or treating obesity without being mixed, and may be administered simultaneously, separately, or sequentially, and may be administered in a single dose or in multiple doses. It is important to administer an amount that may achieve a maximum effect with a minimum amount without causing side effects by considering all of the above-described factors, and this may be easily determined by one of ordinary skill in the art.

The pharmaceutical composition may be administered orally or parenterally, and when administered parenterally, topical application on the skin or intraperitoneal injection, intrarectal injection, subcutaneous injection, intravenous injection, intramuscular injection, intraarterial injection, intramedullary injection, intracardiac injection, intrathecal injection, percutaneous injection, intranasal injection, intraenteric injection, local injection, sublingual injection, or intrathoracic injection may be selected.

The pharmaceutical composition is administered in a pharmaceutically effective amount. The term "pharmaceutically effective amount" refers to an amount sufficient to treat a disease with a reasonable benefit/risk ratio applicable to medical treatment, and an effective dosage level can be determined based on the type and severity of the patient's disease, the activity of the drug, the sensitivity to the drug, administration time, administration route, and excretion rate, treatment duration, simultaneously used drugs, and other factors well known in the medical field.

In one aspect, the pharmaceutical composition may be administered once a day or may be administered in several divided doses. For example, it may be administered every other day or may be administered once a week. Specifically, the pharmaceutical composition may be administered at 0.001 to 1000 mg/kg/day, more specifically 0.1 to 100 mg/kg/day. The administration may be carried out once a day or may be carried out in several divided doses.

Yet another aspect provides a health functional food for preventing or ameliorating obesity, the health functional food including the conjugate.

The "conjugate," "obesity," "prevention," and the like may be within the above-described ranges.

The term "amelioration" may refer to any action that at least reduces a parameter related to the condition being treated, for example, the severity of symptoms. At this time, in order to prevent or ameliorate obesity, the health functional food may be used before or after the onset of the disease, simultaneously with or separately from a drug for treatment.

In the health functional food, an active ingredient may be added to the food as it is or used together with other food or food ingredients, and may be used appropriately according to a conventional method. The mixing amount of the active ingredient may be appropriately determined according to its purpose of use (prevention or amelioration). In general, when manufacturing a food or beverage, the health functional food may be added in an amount of about 15% by weight or less, more specifically about 10% by weight or less, based on the raw materials. However, in the case of long-term intake for the purpose of health and hygiene or health control, the above-mentioned amount may be below the above-mentioned range.

The health functional food may be formulated as one selected from the group consisting of tablets, pills, powder, granules, fine powder, capsules, and liquid formulations, further including one or more of a diluent, an excipient, and an additive. Food to which the compound according to one aspect may be added includes various foods, powder, granules, tablets, capsules, syrups, beverages, gum, tea, vitamin complexes, and health functional foods.

The health functional food, in addition to containing the effective ingredient, may contain other ingredients as essential ingredients without particular limitation. For example, it may contain various flavoring agents or natural carbohydrates as additional ingredients, like a typical beverage. Examples of the above-mentioned natural carbohydrates may include common sugars such as monosaccharides, for example, glucose and fructose; disaccharides, for example, maltose and sucrose; and polysaccharides, for example, dextrin and cyclodextrin, and sugar alcohols such as xylitol, sorbitol, and erythritol. In addition to the above-described flavoring agents, natural flavoring agents (thaumatin, stevia extracts (e.g., rebaudioside A, glycyrrhizin, etc.)) and synthetic flavoring agents (saccharin, aspartame, etc.) may be advantageously used. The proportion of the above-mentioned natural carbohydrates may be appropriately determined by one of ordinary skill in the art.

In addition to the above-mentioned ingredients, the health functional food according to the aspect may contain various nutrients, vitamins, minerals (electrolytes), flavoring agents such as synthetic flavoring agents and natural flavoring agents, coloring agents and thickening agents (cheese, chocolate, etc.), pectic acid and salts thereof, alginic acid and salts thereof, organic acids, protective colloid thickeners, pH regulators, stabilizers, preservatives, glycerin, alcohols, carbonating agents used in carbonated beverages, and the like. These ingredients may be used independently or in combination, and the proportion of these additives may also be appropriately selected by one of ordinary skill in the art.

In one aspect, the health functional food may further include another health functional food for preventing or ameliorating obesity.

The health functional food may be provided as a mixture with the other health functional food for preventing or ameliorating obesity, and the other health functional food for preventing or ameliorating obesity may be health functional food for preventing or ameliorating obesity that is conventionally known or health functional food for preventing or ameliorating obesity that is newly developed.

When the health functional food further includes another health functional food for preventing or ameliorating obesity, or is provided mixed with another health functional food for preventing or ameliorating obesity, it is important to mix an amount that may achieve a maximum effect with a minimum amount without causing side effects, and this may be easily determined by one of ordinary skill in the art.

The health functional food may be consumed in parallel with another health functional food for preventing or ameliorating obesity, and may be consumed simultaneously, separately, or sequentially, and may be consumed in a single dose or in multiple doses. It is important to consume an amount that may achieve a maximum effect with a minimum amount without causing side effects by considering all of the above-described factors, and this may be easily determined by one of ordinary skill in the art.

Yet another aspect provides a method of preventing or treating obesity, the method including a step of administering the conjugate to an individual in need thereof.

The "conjugate," "individual," "administration," "obesity," "prevention," "treatment," and the like may be within the above-described ranges.

The method may be administering in parallel with another pharmaceutical composition for preventing or treating obesity, and the administration may be carried out simultaneously, separately, or sequentially, and may be carried out in a single dose or in multiple doses. It is important to administer an amount that may achieve a maximum effect with a minimum amount without causing side effects by considering all of the above-described factors, and this may be easily determined by one of ordinary skill in the art.

Yet another aspect provides a use of the conjugate for preparing a medicine for preventing or treating obesity.

The "obesity," "prevention," "treatment," "conjugate," and the like may be within the above-described ranges.

### [Advantageous Effects]

It was confirmed that the conjugate and the composition including the same according to one aspect inhibit the hypertrophy of adipocytes, increase the cell membrane expression level of ATP-binding cassette transporter A1 (ABCA1), and reduce the secretion amount of tumor necrosis factor-α (TNF-α). In addition, it was confirmed that the conjugate and composition including the same have a higher blood residual concentration and have better pharmacokinetic properties than glycyrrhizin used alone, and thus can be used in the obesity prevention and/or treatment market/industry.

### [Description of Drawings]

FIG. 1 shows a diagram illustrating the process in which adipose tissue is converted into obese adipose tissue as obesity is induced.
FIG. 2 shows a schematic diagram of the design of peptide candidates formed with the prohibitin (PHB) binding domain of Annexin A2 (ANXA2).
FIG. 3 shows a diagram illustrating an example of utilizing PepSite (results of running PepSite for other adipose tissue targeting sequences).
FIG. 4 shows a diagram illustrating peptide candidates formed with the PHB binding domain of Annexin A2 (ANXA2).
FIG. 5 shows a diagram illustrating the results of running PepSite for peptide candidates.
FIG. 6 shows a diagram illustrating the results of comparing the targeting ability of fluorescein isothiocyanate (FITC)-labeled peptide candidates in adipocytes (3T3-L1) through fluorescence activated cell sorting (FACS).
FIG. 7 shows a diagram illustrating the results of comparing the targeting ability of FITC-labeled peptide candidates in adipocytes (mature 3T3-L1) through FACS.
FIG. 8 shows a diagram illustrating the fluorescence images (scale bar: 25 µm) of FITC-labeled peptide candidates in adipocytes (mature 3T3-L1) obtained using a confocal microscope.
FIG. 9 shows a diagram illustrating the results of comparing the adipocyte targeting ability of 7mer 001 (AHP) and other adipose tissue targeting sequences (ATS) through FACS.
FIG. 10 shows a diagram illustrating the results of confirming the ¹H-nuclear magnetic resonance (NMR) spectra of GL-PEG composites.
FIG. 11 shows a diagram illustrating the results of confirming the Fourier transform infrared (FT-IR) spectra of GL-PEG composites.
FIG. 12 shows a diagram illustrating the results of confirming the matrix-assisted laser desorption/ionization time-of-flight (MALDI-TOF) spectra of GL-PEG composites.
FIG. 13 shows a schematic diagram illustrating the synthesis of a GL-PEG-AHP conjugate.
FIG. 14 shows a diagram illustrating the results of confirming the ¹H-NMR spectrum of the GL-PEG-AHP composite.
FIG. 15 shows a diagram illustrating the results of evaluating the cytotoxicity of drug groups for adipocytes (mature 3T3-L1 cells).
FIG. 16 shows a diagram illustrating the induction of obese adipocytes and the experimental process at the cell experiment level.
FIG. 17 shows a diagram illustrating the results of comparing the difference in fat droplet size in adipocytes according to free fatty-acid (FFA) treatment (x 200).
FIG. 18 shows a diagram illustrating the results of comparing the anti-hypertrophic effect in obese adipocytes according to the timing of drug treatment (x 200).
FIG. 19 shows a diagram illustrating the results of comparing the anti-hypertrophic effect according to the drug concentration on obese adipocytes (FFA-treated mature 3T3-L1 cells) during drug pretreatment (%, n=5).
FIG. 20 shows a diagram illustrating the results of comparing the quantitative anti-hypertrophic effects of the drug pretreatment and co-treatment groups in obese adipocytes (FFA-treated mature 3T3-L1 cells) (%, n=5).
FIG. 21 shows a schematic diagram illustrating the immune cell stimulation mechanism of obese adipocytes and the co-culture model.
FIG. 22 shows a diagram illustrating a schedule for creating a co-culture model.
FIG. 23 shows a diagram illustrating the results of measuring the tumor necrosis factor-α (TNF-α) secretion amount of obese adipocytes (co-cultured with FFA-treated mature 3T3-L1, A) and immune cells (co-cultured with Raw 264.7 cells, B) in a co-culture cell environment simulating obese adipose tissue (n=5, TNF-α ELISA).
FIG. 24 shows a diagram illustrating the results of measuring the cell number of the co-cultured immune cells (co-cultured with Raw 264.7 cells) (%, n=5).
FIG. 25 shows a schedule for the experiment to confirm the expression level of ATP-binding cassette transporter A1 (ABCA1) in the cell membrane (A) and a schematic diagram illustrating the process of promoting ABCA1 cell membrane expression by glycyrrhizin (B).
FIG. 26 shows a diagram illustrating the results of confirming ABCA1 expression in the adipocyte (mature 3T3-L1) cell membrane after drug treatment through immunofluorescence staining.
FIG. 27 shows a schematic diagram illustrating the mechanism of action of the liver-X-receptor (LXR) inhibitor GSK2033.
FIG. 28 shows a diagram illustrating the results of confirming ABCA1 expression in the adipocyte (mature 3T3-L1) cell membrane after drug treatment through immunofluorescence staining confirmed according to GSK2033 treatment.
FIG. 29 shows a diagram illustrating the blood drug residual concentration when GL and GL-PEG-AHP were injected intraperitoneally in C57BL/6J animals.
FIG. 30 shows a schematic diagram illustrating the composition and mechanism of action of GL-PEG-AHP and its anti-inflammatory effect.

### [Modes of the Invention]

Hereinafter, the present invention will be described in more detail through examples. However, these examples are intended to exemplify the present invention, and the scope of the present invention is not limited to these examples.

### Examples

### 1. Derivation of candidates for discovery of new adipocyte targeting peptides

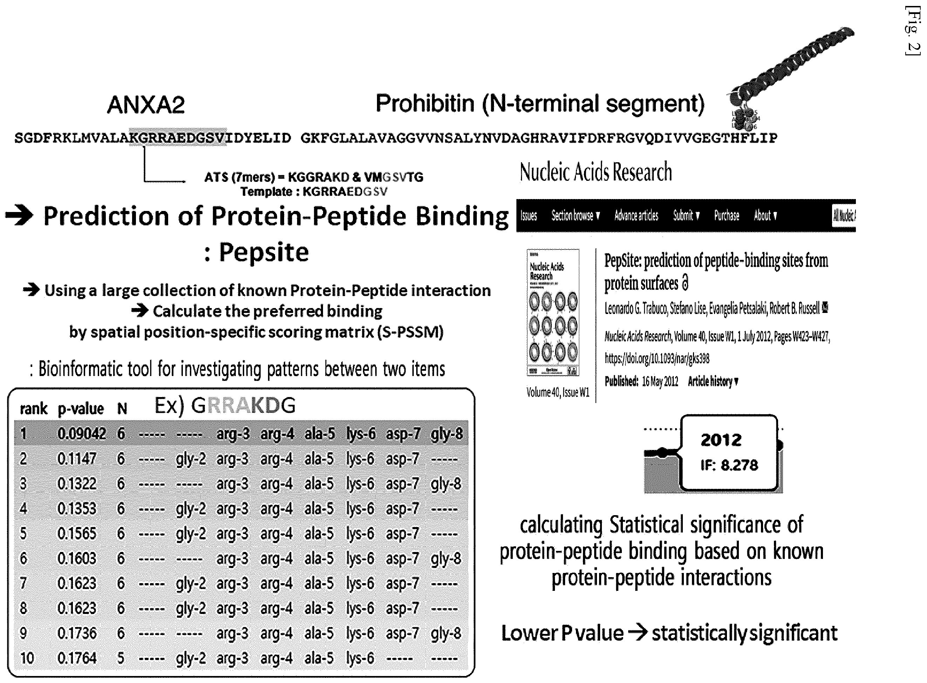

A bioinformatics approach was used to derive candidates for discovering new adipocyte-targeting peptides. The prohibitin (PHB)-binding domain of Annexin A2 (ANXA2), which is another membrane protein that forms a complex with PHB, a membrane protein specifically expressed on the surface of adipocytes, was investigated to establish a template. The template was KGRRAEDGSV (SEQ ID NO: 2). FIG. 2 shows a schematic diagram illustrating the design of peptide candidates formed from the PHB-binding domain of ANXA2. Specifically, the PHB-binding site of the ANXA2 protein was used as a template, and the working sequence was assumed to be 5mer and 7mer, and the candidates were selected by fragmentation (Table 1).

**[Table 1]**

| **5mer** | **SEQ ID NO.** | **7mer** | **SEQ ID NO.** |
|---|---|---|---|
| KGGRA | SEQ ID NO: 3 | KGGRAKR | SEQ ID NO: 11 |
| KGRRA | SEQ ID NO: 4 | KGGRAKK | SEQ ID NO: 12 |
| GGRAK | SEQ ID NO: 5 | KGGRAKH | SEQ ID NO: 13 |
| GRRAK | SEQ ID NO: 6 | KGRRAKD | SEQ ID NO: 14 |
| GRAKD | SEQ ID NO: 7 | KGRRAKR | SEQ ID NO: 15 |
| RRAED | SEQ ID NO: 8 | KGRRAKK | SEQ ID NO: 16 |
| RAEDG | SEQ ID NO: 9 | KGRRAKH | SEQ ID NO: 17 |
| KDGSV | SEQ ID NO: 10 | KGGRAED | SEQ ID NO: 18 |
| | | GRRAKDG | SEQ ID NO: 19 |
| | | RRAEGSV | SEQ ID NO: 20 |
| | | RAKDGSV | SEQ ID NO: 21 |

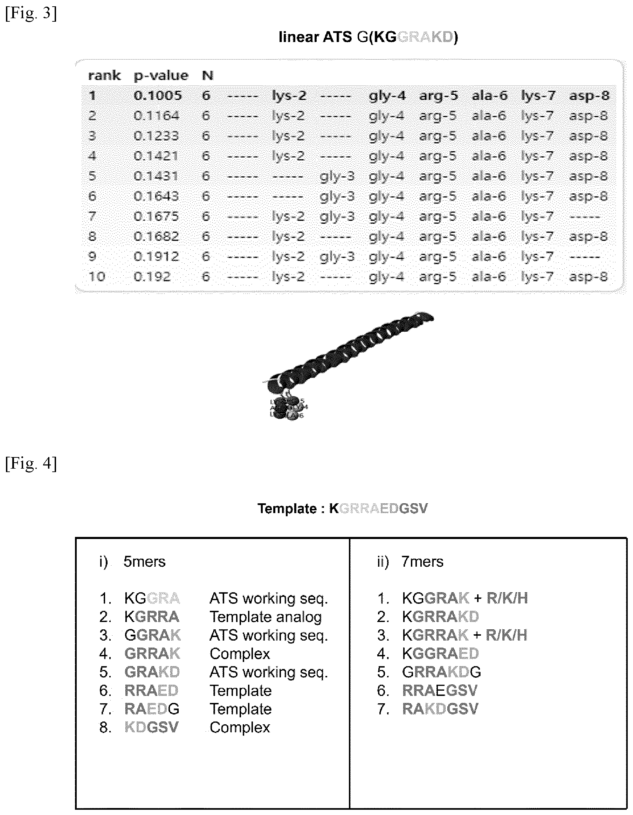

PepSite, a program that predicts the binding of peptides and proteins, was employed to measure the p-value of each candidate peptide. FIG. 3 shows an example of utilizing PepSite and p-values obtained by running the existing sequence ATS on PepSite, confirming that that the lowest p-value value was 0.1. In addition, FIG. 4 shows the peptide candidates derived based on the template. PepSite was run separately for two types of candidates: 7mer candidates considering that the working region of the existing sequence is 7mer; and 5mer candidates based on the hypothesis that there may be a compressed working region of the existing sequence. As a result, it was confirmed that the peptide candidates were divided into three major working regions: GRRA, KD, and GSV. Since a lower p-value indicates a more significant protein-peptide binding ability, the three groups with the lowest p-values were ultimately selected as the initial candidates for conducting experiments in actual adipocytes.

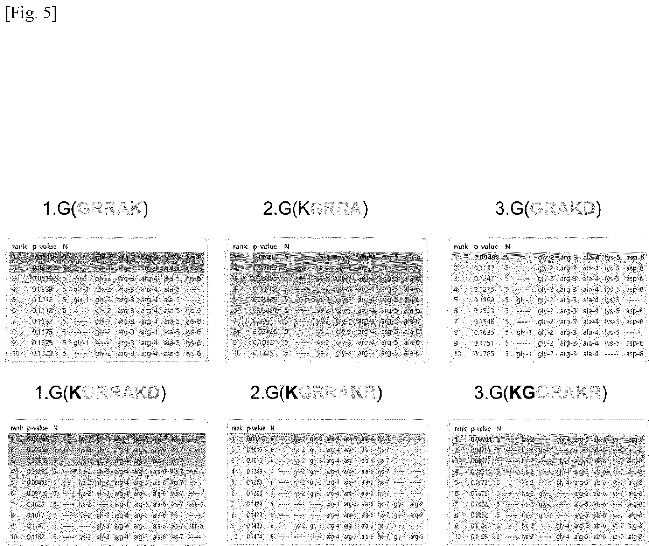

In addition, FIG. 5 shows the results of running PepSite for the peptide candidates, and the three sequences that were predicted to have statistically significant binding to PHB with the lowest p-values were each selected from the 5mer candidates and the 7mer candidates. Based on the two sequences G (GRRAK: SEQ ID NO: 6) and G (KGRRAKD: SEQ ID NO: 14) with the lowest p-values among the 5mer candidates and the 7mer candidates, it was estimated that the GRRAK sequence binds to PHB most significantly based on the bioinformatic analysis results.

### 2. Differentiation process of preadipocytes to adipocytes

To conduct cell experiments in adipocytes, preadipocytes were differentiated into adipocytes. Specifically, 3T3-L1 preadipocytes were first seeded in a well plate of a size suitable for the experimental purpose, and then cultured to 80% to 90% confluence. Thereafter, the cells were treated with an adipocyte differentiation induction reagent (insulin 10 µg/ml, dexamethasone 0.4 µg/ml, 3-isobutyl-1-methylxanthine 111 µg/ml) for three days. After washing the adipocyte differentiation induction reagent with phosphate-buffered saline (PBS), the cells were treated with Dulbecco's modified eagle medium (DMEM) containing insulin (10 µg/ml) every two days, and differentiation was induced for up to 14 days to generate adipocytes. Thereafter, on day 14 of the adipocyte differentiation induction, the cells were fixed with 4% paraformaldehyde and then treated with the Oil red O staining solution for two hours. In addition, the staining solution accumulated inside the cells was eluted with isopropanol, and a microplate reader was used to verify whether fatty acids were accumulated inside the adipocytes at a wavelength of 510 nm to finally confirm the differentiation.

### 3. Selection of derived candidate peptides through fluorescence-activated cell sorting (FACS)

The targeting effect of the above-described candidate peptides was quantitatively verified in actual adipocytes and preadipocytes. Specifically, differentiated 3T3-L1 preadipocytes were treated at 37 °C for 30 minutes with 1 µM of the final FITC-labeled candidate peptides dissolved in DMEM. The final FITC-labeled candidate peptides are shown in Table 2.

**[Table 2]**

| **5mer** | **SEQ ID NO.** | **7mer** | **SEQ ID NO.** |
|---|---|---|---|
| KGRRA | SEQ ID NO: 4 | KGGRAKR | SEQ ID NO: 11 |
| GRRAK | SEQ ID NO: 6 | KGRRAKD | SEQ ID NO: 14 |
| GRAKD | SEQ ID NO: 7 | KGRRAKR | SEQ ID NO: 15 |

After washing with PBS, cells were detached from the well plate using a trypsin-ethlyenediaminetetraacetic acid (EDTA) (0.5%) solution and centrifuged at 1100 rpm for three minutes to obtain sedimented cells. Thereafter, cells were suspended in 0.8 ml of PBS in a glass container for FACS equipment, and the difference in targeting effect among the candidates was confirmed using the FACS equipment.

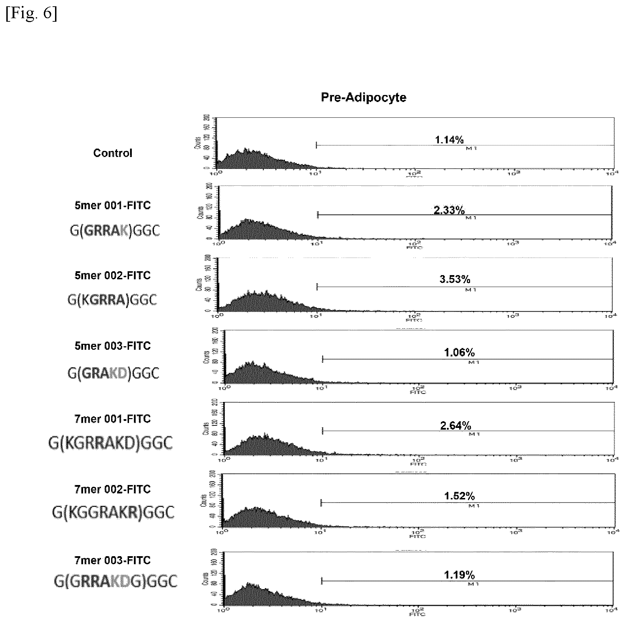

FIG. 6 shows the results of comparing the targeting ability of six actual peptide candidates in preadipocytes through FACS. Specifically, since preadipocytes are known to not express PHB, which is targeted by the adipocyte targeting sequence, it was confirmed that none of the six sequences had targeting ability compared to the control group.

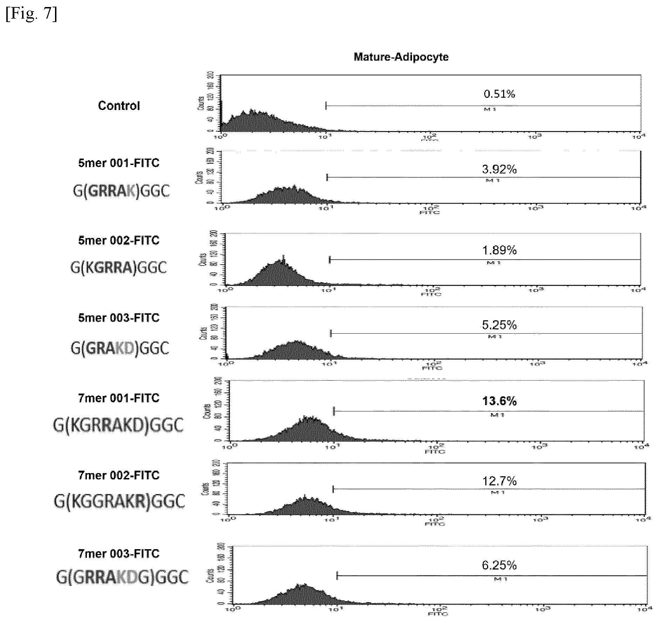

In addition, FIG. 7 shows the results of comparing the targeting ability of the actual six peptide candidates in adipocytes (mature 3T3-L1) through FACS. Specifically, it is well known that adipocytes express PHB on the cell surface, unlike the preadipocytes shown in FIG. 6. Therefore, it was confirmed that each candidate peptide had a different targeting ability, as shown in the above-described results. In addition, unlike the previous bioinformatic analysis, it was confirmed that the working region GRRAK with the lowest p-value did not have a significant targeting ability in actual adipocytes. In addition, it was confirmed that the peptide sequence having the 7mer KGRRAKD as a working region, exhibited the lowest p-value among the tested 7-mer candidates, and also demonstrated the most effective targeting ability.

### 4. Confirmation of binding of the derived candidate peptides to adipocyte outer membrane using fluorescence images

The binding of the candidate peptides to the adipocyte outer membrane was confirmed through fluorescence imaging. Specifically, differentiated 3T3-L1 preadipocytes were treated with 1 µM of the finally selected FITC-labeled candidate peptides in Table 2 dissolved in DMEM for 30 minutes at 37 °C. After washing with PBS, cell nuclei were stained using 4'6,-diamidino-2-phenylindole (DAPI). Fluorescence images were taken using a confocal microscope (green fluorescence (FITC) = 488 nm, blue fluorescence (DAPI) = 405 nm).

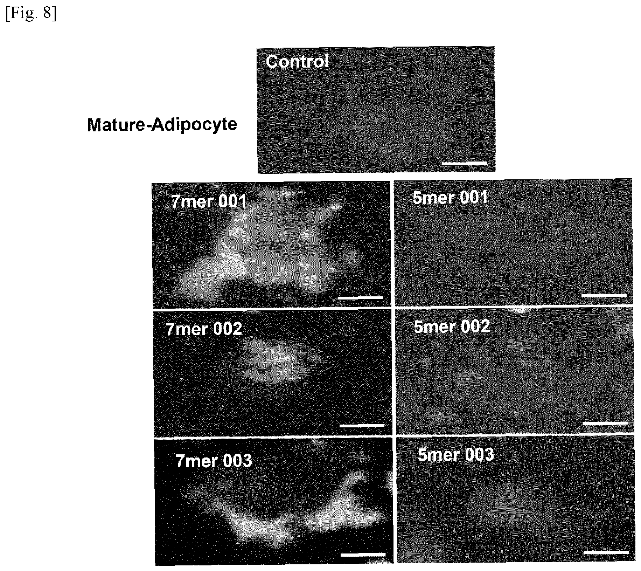

As a result, it was confirmed that the 7mer 001 (AHP) sequence had the best adipocyte targeting ability, which was consistent with the above-described results obtained through the FACS experiment of Example 3 (FIG. 8).

### 5. Comparison of adipocyte targeting effect of the finally selected peptide (adipose tissue homing peptide: AHP) with the known adipose tissue targeting sequence (adipose targeting sequence: ATS) through FACS

The finally selected candidate peptide (adipose tissue homing peptide: AHP) (SEQ ID NO: 14: KGRRAKD) was quantitatively compared with another adipose tissue targeting sequence (adipose targeting sequence: ATS) (SEQ ID NO: 22: KGGRAKD) in adipocytes. Specifically, differentiated 3T3-L1 preadipocytes were treated with 1 µM FITC-labeled AHP and ATS dissolved in DMEM for 30 min at 37 °C. After washing with PBS, the cells were detached from the well plate using a trypsin-EDTA (0.5%) solution and centrifuged at 1100 rpm for three min to obtain sedimented cells. Thereafter, the cells were resuspended in 0.8 ml of PBS in a glass container for FACS equipment, and the difference in targeting effect among the candidates was confirmed using the FACS equipment.

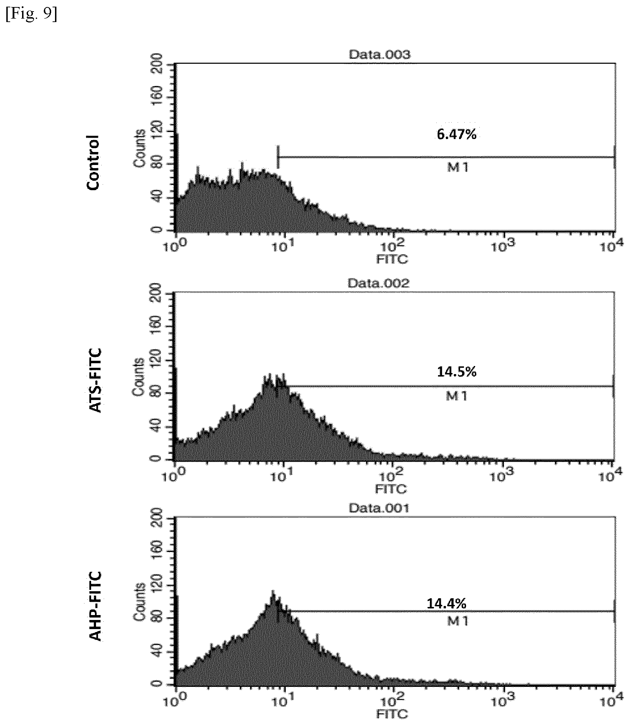

As a result, it was confirmed that the peptide 7mer 001, which was finally selected based on the bioinformatic analysis, was not inferior in efficacy to ATS (FIG. 9). The above-described results suggest the possibility that existing sequences can be modified and even improved through a bioinformatics approach.

### 6. GL-PEG conjugation

A carboxyl group (COOH) of glycyrrhizin (GL) was conjugated with an amine group (NH₂) of thiol-polyethylene-glycol-amine (PEG). Specifically, 26 mg of glycyrrhizin was dissolved in 20 ml of deionized water, and the pH was adjusted to 6. 50 mg of 1-ethyl-3-(3-dimethylaminopropyl)carbodiimide (EDC) was dissolved, and after 15 minutes, 16 mg of N-hydroxysuccinimide (NHS) was dissolved, and then the resulting mixture was allowed to react at room temperature (RT) for one hour. In addition, 16 mg of PEG was dissolved in the solution, and the mixture was allowed to react in the dark for four hours at room temperature. After dialysis using a 2,000 molecular weight cut-off (MWCO) centricon, the product was cooled in a -80 °C deep freezer and then freeze-dried to obtain GL-PEG in a powder form.

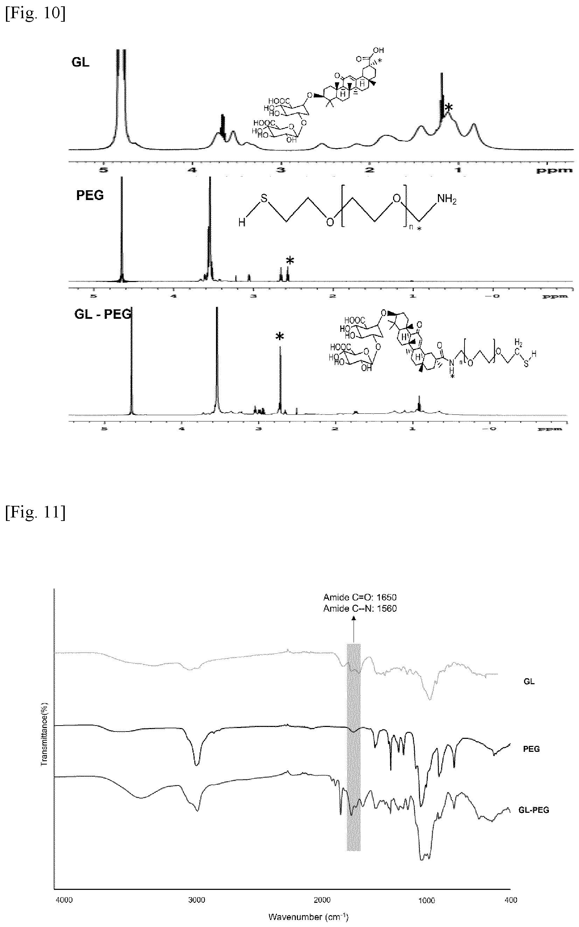

The ¹H-NMR spectrum of the GL-PEG conjugate was confirmed, and the results are shown in FIG. 10. Specifically, the ¹H-NMR results showed that the H-C-N peak of PEG disappeared around 2.5 ppm in the GL-PEG conjugate, the amine bond peak of GL-PEG was observed around 2.7 ppm, and the C-C-H peak of glycyrrhizin was confirmed around 1.2 ppm.

The Fourier transform infrared (FT-IR) spectrum of the GL-PEG conjugate was confirmed, and the results are shown in FIG. 11. Specifically, it was confirmed, in the GL-PEG, that not only were the unique peaks of GL and PEG characteristically observed, but also the peaks at 1650 cm⁻¹ and 1550 cm⁻¹, known as amine bonds, were prominently observed.

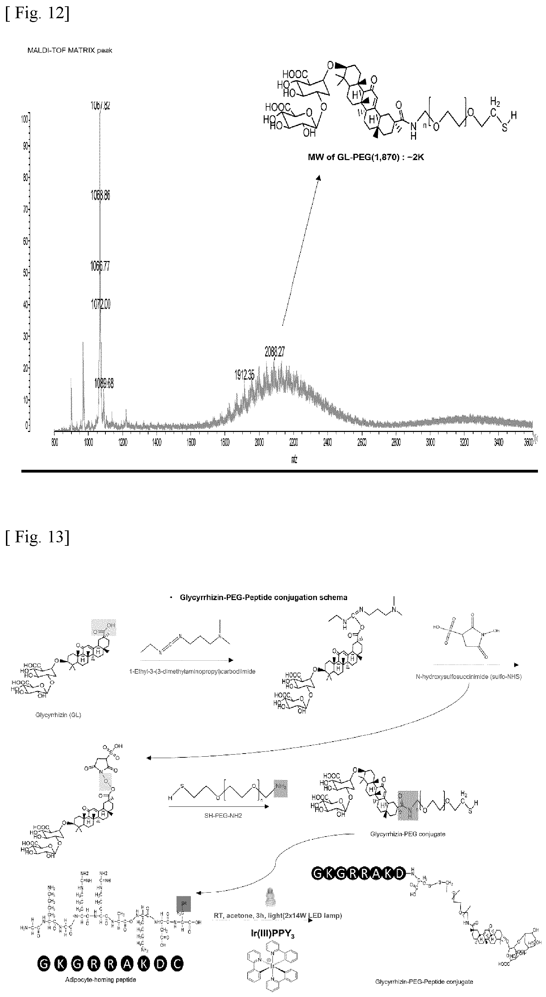

In addition, the matrix-assisted laser desorption/ionization time-of-flight (MALDI-TOF) spectrum of the GL-PEG conjugate was confirmed, and the results are shown in FIG. 12. Specifically, the sum of the molecular weights of GL and PEG is 871 + 1,000 = 1,871, but it was confirmed that the a MALDI-TOF peak that occupied most of the area actually appeared around 2,000.

### 7. GL-PEG-AHP conjugation

The thiol (SH) groups of GL-PEG and AHP (SEQ ID NO: 1: GKGRRAKDC) obtained through Example 6 were conjugated. FIG. 13 shows a schematic diagram illustrating the chemical bond between the carboxyl group (-COOH) of GL and the amine part of PEG using the 1-ethyl-3-(3-dimethylaminopropyl) carbodiimide/N-hydroxysuccinimide (EDC/NHS) method and the disulfide bond formation between the thiol group (-SH) of GL-PEG and the thiol group (-SH) of AHP using the tris(2-phenylpyridine)iridium (III) (Ir(ppy)₃) catalyst.

Specifically, for the synthesis process using Ir(ppy)₃, 4 mg of GL-PEG was dissolved in 6 ml of 70% acetone, and then a solution containing 1 mg of AHP dissolved in 70% acetone was slowly added dropwise. 6.5 µg of Ir(ppy)₃ catalyst was added, and a photoreaction was performed for three hours under the condition that 500 lumens of light was present at a distance of 10 cm. After the reaction was completed, the light source was removed, and the vaporization of acetone was induced in a hood overnight. After cooling in an ultra-low temperature freezer at -80 °C, the product was freeze-dried to obtain GL-PEG-AHP in a powder form. At this time, the Ir(ppy)₃ catalyst was introduced to increase the efficiency of disulfide bond formation through a radical reaction via photoreaction.

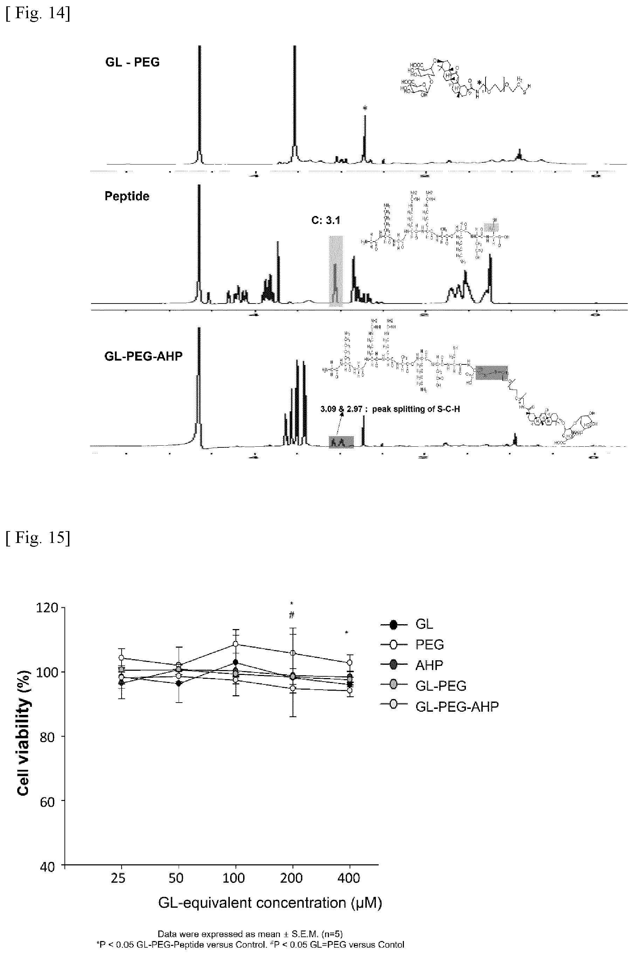

The ¹H-NMR spectrum of the GL-PEG-AHP conjugate was confirmed, and the results are shown in FIG. 14. Specifically, since the thiol peaks (-SH) of both GL-PEG and AHP were around ~1.0 and thus were difficult to analyze, the formation of a disulfide bond was indirectly confirmed using the fact that the S-C-H peak appears between 2.5 and 3.5. In summary, it is known that when a disulfide bond is formed, the number of hydrogens facing the hydrogen of S-C-H and the electronic stability change, causing the existing peak to fragment. It was confirmed that this phenomenon was characteristically shown in the H-NMR results of the GL-PEG-AHP conjugate.

### 8. Evaluation of cytotoxicity of GL derivatives in adipocytes

To determine whether GL derivatives are toxic to 3T3-L1 preadipocytes, adipocytes cultured in 96-well plates were treated with each group (control, GL, PEG, AHP, GL-PEG, and GL-PEG-AHP) at various concentrations (25 µM, 50 µM, 100 µM, 200 µM, and 400 µM) for 24 h. All groups were washed twice with PBS and treated with 100 µl of DMEM + 10 µl of Cell Counting Kit-8 (CCK) solution. After culturing the cells in foil for two hours, the absorbance was measured at 450 nm using a microplate reader. As a result, it was confirmed that no significant cytotoxicity was exhibited up to a GL-equivalent concentration of 400 µM (FIG. 15).

### 9. In vitro obese adipocyte induction process using saturated fatty acids

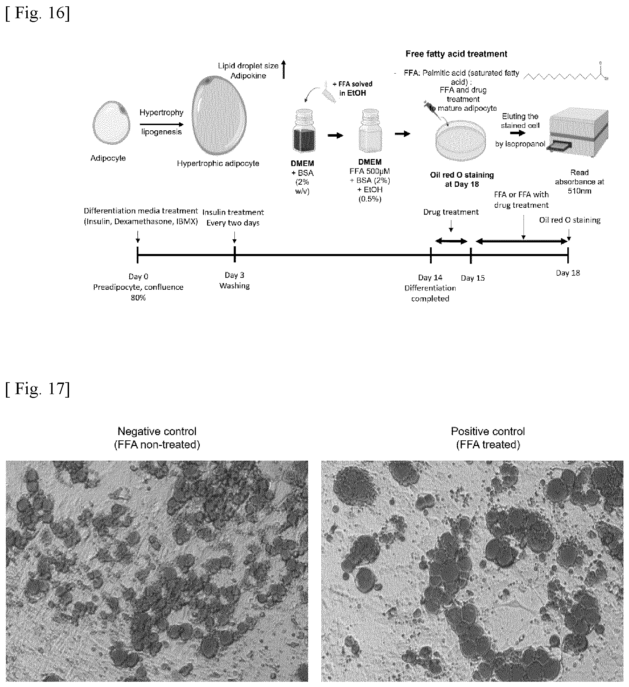

To induce obesity in adipocytes at the cell experiment level, cells were treated with saturated fatty acids, and the process of inducing obese adipocytes and performing the experiment at the cell experiment level are shown in FIG. 16. Specifically, a saturated fatty acid (free fatty-acid) (FFA, palmitic acid) was dissolved in a 2% w/v bovine serum albumin (BSA) and 0.5% ethanol DMEM solution to a concentration of 500 µM. After treating with FFA for three days, the cells were washed, subjected to an Oil Red O staining solution process, and then the amount of accumulated fatty acids was measured through the absorbance at 510 nm. The results are shown in FIG. 17, and it was confirmed even through qualitative observation that the size of single fat droplets was very different. Considering that not only the accumulated amount of fat but also the size of the fat droplet is a very important indicator of insulin resistance, it was confirmed that the process of inducing obese adipocytes using saturated fatty acids was successfully performed.

### 10. Measurement of anti-hypertrophic effect according to the concentration of GL derivatives

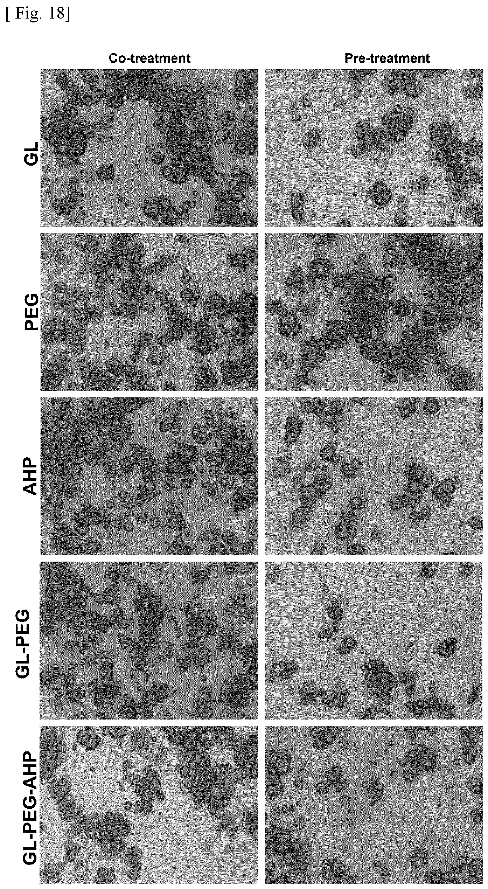

The anti-hypertrophy effect of GL derivatives in obesity-induced adipocytes according to the concentration was confirmed at the cell experiment level. In the above-described obese adipocyte induction process, cells were treated with each group (control, GL, PEG, AHP, GL-PEG, and GL-PEG-AHP) at various concentrations (25 µM, 50 µM, 100 µM, 200 µM, 400 µM) on day 14 of differentiation, one day before FFA treatment, and then treated with FFA for three days until day 18 of differentiation to induce an obesity model. The amount of fatty acids accumulated inside the cells was measured through the Oil Red O staining process on day 18 of differentiation. As a result, the case where adipocytes (mature 3T3-L1 cells) were treated with a drug 24 hours before fatty acid treatment and the case where the adipocytes were treated with the drug and the fatty acid at the same time were qualitatively compared, and it was confirmed that the size of the adipocytes was reduced more significantly in the drug pre-treatment group (FIG. 18). These results showed that in the anti-hypertrophy mechanism of glycyrrhizin is a significant variable that induces a temporal difference that may affect the adipocyte hypertrophy process in advance by increasing the expression of the ATP-binding cassette 1 (ABCA1) transporter. Therefore, an experiment to confirm the expression level of the ABCA1 transporter was further designed.

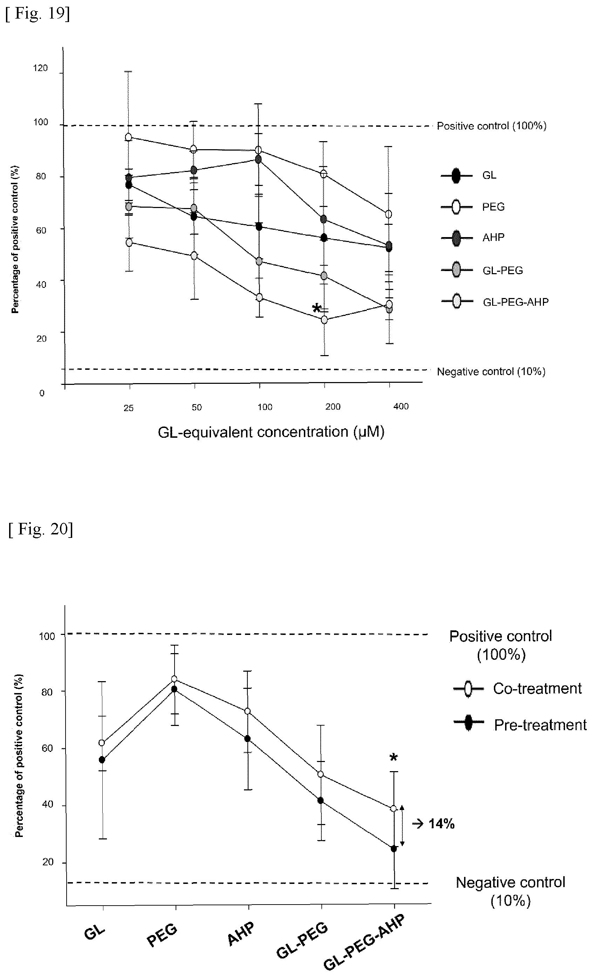

In addition, as a result of confirming the anti-hypertrophic effect on obese adipocytes (FFA-treated mature 3T3-L1 cells) during drug pretreatment according to the drug concentration (25 µM, 50 µM, 100 µM, 200 µM, 400 µM), the group that exhibited the most effective anti-hypertrophic effect on the obese adipocytes (FFA-treated mature 3T3-L1 cells) was the GL-PEG-AHP group at all concentrations, and the next most effective group was GL-PEG (FIG. 19). In order to effectively perform further experiments, subsequent experiments were conducted at a GL-equivalent concentration of 200 µM, which exhibited the best anti-hypertrophic effect and a significant difference in anti-hypertrophic effect between the groups.

### 11. Measurement of anti-hypertrophic effects of GL derivatives according to fatty acid treatment method

It was confirmed whether the anti-hypertrophic effect according to the expected mechanism of GL derivatives differed depending on the fatty acid treatment method. Specifically, in the above-described process of inducing obese adipocytes, cells were treated with each group (control group, GL, PEG, AHP, GL-PEG, and GL-PEG-AHP) on day 14 of differentiation, one day before FFA treatment. On day 15 of differentiation, after the medium was washed (the drug was washed out for the drug pretreatment group), cells were treated with the experimental groups together with FFA or treated only with FFA at different drug treatment time points. Cells were treated with FFA for three days, and the obese model was induced until the day 18 of differentiation. On day 18 of differentiation, the amount of fatty acids accumulated inside the cells was measured through the Oil Red O staining process.

As a result, it was confirmed that the same tendency as the previously confirmed qualitative cell photographs was exhibited, and it was also confirmed that the difference in drug efficacy was particularly prominent in the GL derivative groups between pretreatment and co-treatment (FIG. 20). The group that exhibited the greatest efficacy difference was the GL-PEG-AHP group, and it was inferred that the mechanism of the anti-hypertrophic effect (ABCA1 transporter and anti-hypertrophy related mechanism) was best exhibited in the GL-PEG-AHP group.

### 12. Preparation of co-culture model of obese adipocytes and immune cell lines

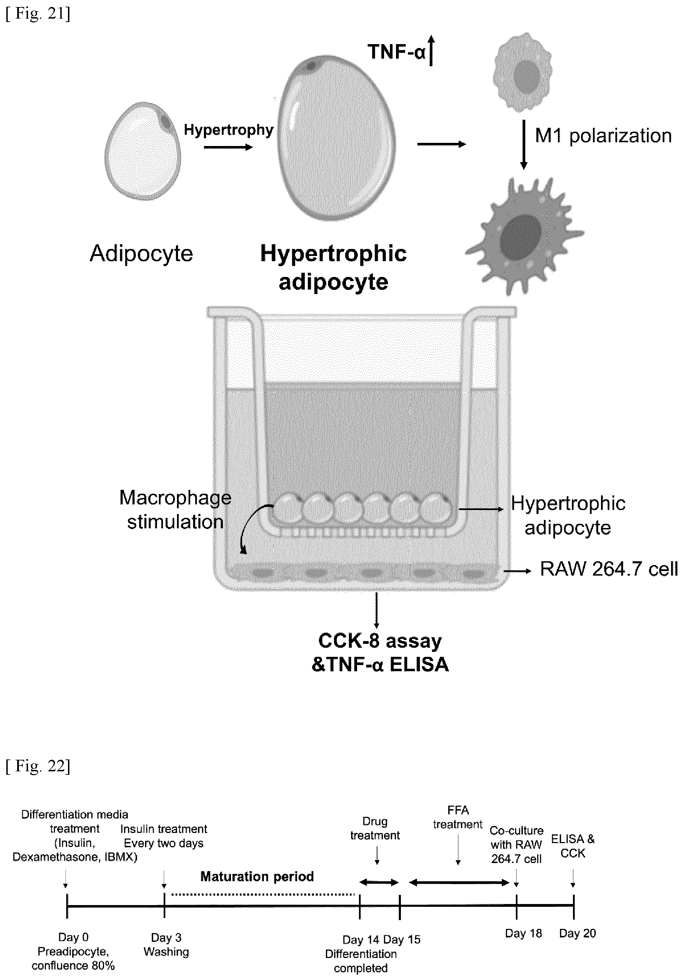

To mimic the cellular environment of obese adipose tissue, a co-culture model was established. FIG. 21 shows a schematic diagram illustrating the immune cell stimulation mechanism of obese adipocytes and the co-culture model, and FIG. 22 shows a schedule for creating the co-culture model. Specifically, the obese adipocyte model was induced inside a Transwell 24-well plate (an insert with a pore size of 8.0 µm). On day 17 of differentiation, 0.5 x 10⁵ RAW 264.7 cells (immune cells) were seeded in the Transwell 24-well plate. On day 18 of differentiation, co-culture was performed for 48 hours.

### 13. Measurement of TNF-α secretion amount in obese adipocytes

The TNF-α secretion amount in obese adipocytes was confirmed. The obese adipocyte model was treated with each group (control, GL, PEG, AHP, GL-PEG, and GL-PEG-AHP) for 24 hours one day before treatment with FFA. Thereafter, the cells were inserted into the interior of the above-mentioned co-culture model of obese adipocytes and immune cells and cultured with Raw 264.7 cells. On day 2 of co-culture, the medium from the obese adipocytes and the Raw 264.7 cells was obtained, and the amount of TNF-α in the medium was measured using a TNF-α enzyme-linked immunosorbent assay (ELISA).

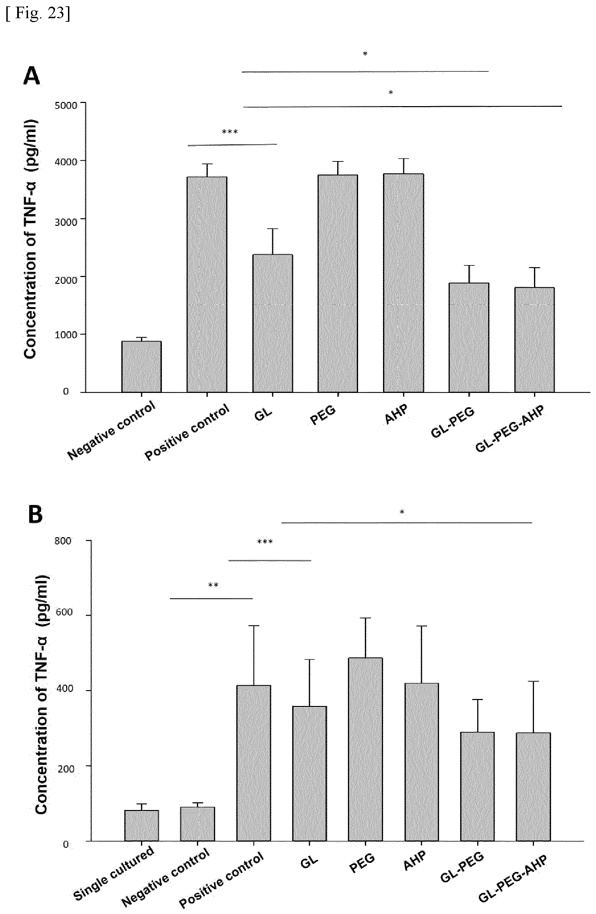

As a result, it was confirmed that the TNF-α secretion amount for each of the two groups was significantly reduced in the GL derivative-treated groups in both the obese adipocytes (FIG. 23A) and the immune cells (FIG. 23B). In particular, the TNF-α secretion amount did not significantly increase when the immune cells were co-cultured with normal adipocytes, whereas the secretion amount significantly increased when they were co-cultured with the obese adipocytes, confirming that the cell experiment mimicking obese adipose tissue was performed well and that GL derivatives had an inhibitory effect thereon.

### 14. Measurement of cell division ability of immune cells stimulated by obese adipocytes

The cell division ability of immune cells with an inflammatory phenotype stimulated by obese adipocytes was measured. Specifically, the obese adipocyte model was treated with each group (control, GL, PEG, AHP, GL-PEG, and GL-PEG-AHP) for 24 hours one day before treating FFA. Thereafter, the cells were inserted into the interior of the above-mentioned co-culture model of obese adipocytes and immune cells and cultured with Raw 264.7 cells. On day 2 of co-culture, all groups of Raw 264.7 cells were washed twice with PBS and treated with 100 µl of DMEM + 10 µl of CCK solution. After culturing the cells in foil for two hours, the absorbance was measured at 450 nm using a microplate reader.

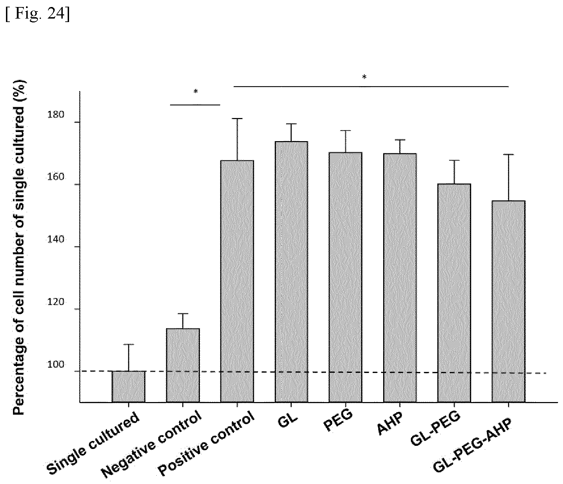

As a result, it was confirmed that the immune cell proliferation ability significantly increased in terms of cell number when the immune cells were co-cultured with obese adipocytes (FIG. 24). In this experiment, the GL derivatives did not significantly reduce the proliferation ability, but when compared to the previous TNF-α secretion experiment, it was inferred that the ability to reduce inflammatory cytokine secretion was not a physical property change caused by the decrease in cell number, but that the GL derivatives caused a chemical change in the internal mechanism of the cells to promote this change.

### 15. Fluorescence image confirmation of ABCA1 transporter cell membrane expression by GL derivatives in adipocytes

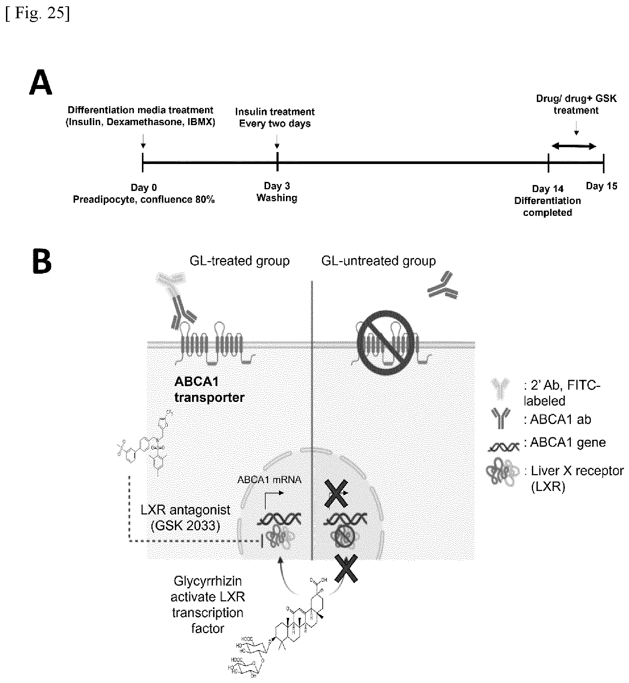

The cell membrane expression level of ABCA1 in adipocytes by GL derivatives was confirmed through fluorescence imaging. FIG. 25 shows the schedule (A) and schematic diagram (B) of the mechanism for the experiment to confirm the promotion of ABCA1 expression by GL. Specifically, 4 x 10⁴ adipocytes were seeded in a 24-well plate and treated with each group (control, GL, PEG, AHP, GL-PEG, and GL-PEG-AHP) for 24 hours on day 14 of adipocyte differentiation. The cells were fixed using 4% paraformaldehyde (PFA). After washing with PBS, they were incubated with 10% goat serum and 0.02% Tween-20 for 30 minutes at room temperature. Cells were treated with the ABCA1 antibody (Host: rabbit, 1:300) in phosphate buffered saline with Tween 20 (PBST) at room temperature for two hours. After washing with PBST, the cells were treated with an FITC-labeled secondary antibody (goat-anti-rabbit, 1:500) at room temperature for one hour. After washing with PBS, the cells were treated with DAPI for one minute at room temperature, and then fluorescence images were obtained.

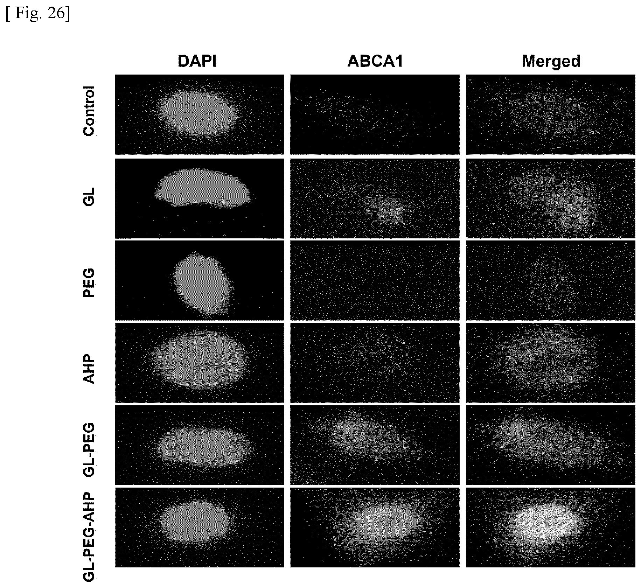

As a result, it was confirmed that the cell membrane expression level of ABCA1 significantly increased in the GL derivative groups (FIG. 26). These results are consistent with the experimental results to date and show the context of the hypothesized effect of ABCA1 on suppressing the hypertrophy of adipocytes due to its cholesterol efflux function.

### 16. Fluorescence image confirmation of ABCA1 cell membrane expression by GL derivatives in adipocytes treated with liver-X-receptor (LXR) inhibitor

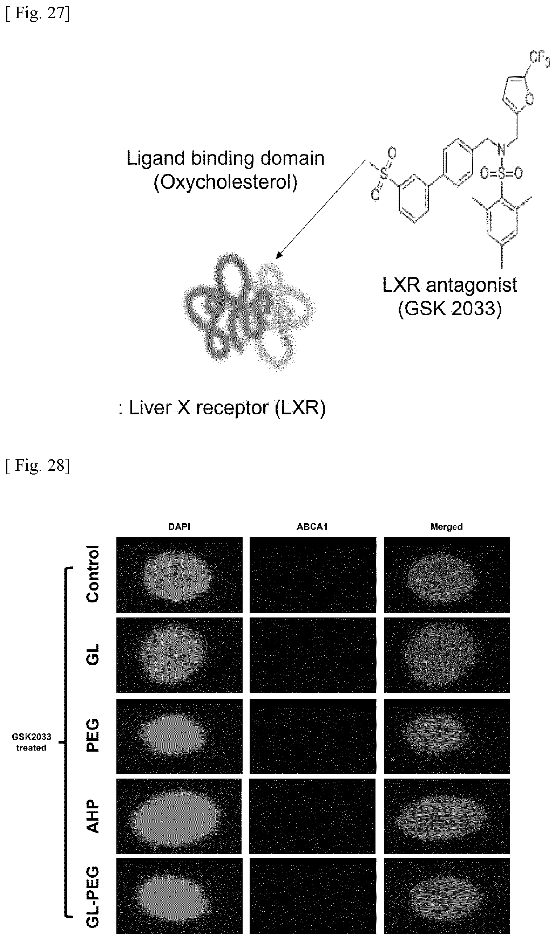

It was confirmed through fluorescence images whether the cell membrane expression level of ABCA1 by GL derivatives is reduced in adipocytes by GSK2033, an LXR inhibitor. FIG. 27 shows a schematic diagram of the mechanism of action of the LXR inhibitor GSK2033. Specifically, 4 x 10⁴ adipocytes were seeded in a 24-well plate and treated with each group (control, GL, PEG, AHP, GL-PEG, and GL-PEG-AHP) for 24 hours on day 14 of adipocyte differentiation. The cells were fixed using 4% PFA. After washing with PBS, the cells were incubated with 10% goat serum and 0.02% Tween-20 for 30 minutes at room temperature. The cells were treated with the ABCA1 antibody (Host: rabbit, 1:300) in PBST at room temperature for two hours. After washing with PBST, the cells were treated with an FITC-labeled secondary antibody (goat-anti-rabbit, 1:500) at room temperature for one hour. After washing with PBS, the cells were treated with DAPI for one minute at room temperature, and then fluorescence images were obtained.

As a result, it was confirmed that the expression of ABCA1 was suppressed in GL derivatives as well by the GSK2033 treatment (FIG. 28), and it was inferred from these results that the expression of ABCA1 by GL derivatives occurred through the activation of LXR.

### 17. Pharmacokinetic measurements of GL derivatives

The pharmacokinetic indices were calculated by measuring the blood residual concentrations of GL derivatives. Specifically, GL and GL-PEG-AHP were injected intraperitoneally in an amount of 13.5 mg/kg into animals (C57BL/6J, 6 weeks old, 5 per cage). At each time point (0, 20, 30, 120, 240, and 480 minutes) following the intraperitoneal injection, the animals were sacrificed, and 300 µl of blood samples were obtained through the abdominal vein. The obtained blood samples were placed in EDTA tubes and centrifuged at 824 ×g for 30 minutes at 4 °C. 50 µl of the supernatant was mixed with 100 µl of methanol, and the resulting mixture was vortexed for 10 minutes. Thereafter, centrifugation was performed again at 10,000 ×g for 10 minutes. 100 µl of the supernatant was mixed with 900 µl of the mobile phase, and the resulting mixture was filtered through a 0.45 µm syringe filter, and HPLC was performed (HPLC column: C8 column; mobile phase = methanol:acetonitrile:water:acetic acid = 55: 23.7: 19.2: 0.68; flow rate: 1 ml/min; injection volume: 20 µl) to measure the blood residual concentration of the drug over time. Detection was performed through UV at 245 nm.

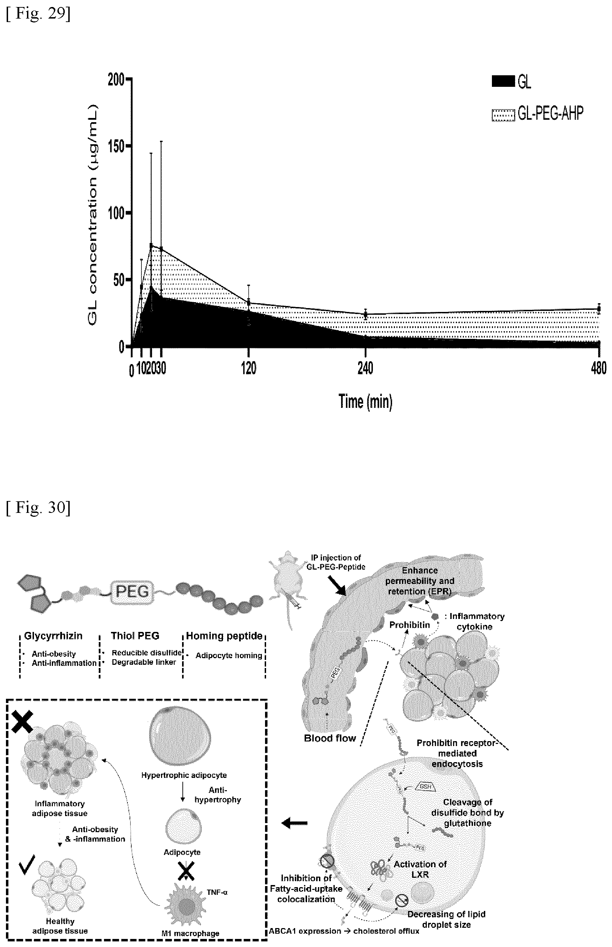

The numerical values of the blood retention concentration determined by collecting blood samples at each timepoint after intraperitoneal injection are presented in a graph (FIG. 29), and it was confirmed that, the GL-PEG-AHP group exhibited a blood retention concentration that was approximately 2.3 times that of the GL group (Table 3).

**[Table 3]**

| **Parameters** | **Cₘₐₓ (µg/ml)** | **AUCₗₐₛₜ (µg/ml/ml)** | **Tₘₐₓ (min)** | **T_{1/2} (hour)** |
|---|---|---|---|---|
| GL | 43.8 | 6866 | 20 | 3.3 |
| GL-PEG-AHP | 75.2 | 15977 | 20 | 3.45 |

Finally, FIG. 30 shows a schematic diagram illustrating the composition, the mechanism of action, and the anti-inflammatory effect of GL-PEG-AHP prepared in the present invention.

## Claims

1. A peptide consisting of an amino acid sequence of SEQ ID NO. 1.

2. The peptide of claim 1, wherein the peptide includes a prohibitin (PHB) targeting sequence of Annexin A2 (ANXA2).

3. A conjugate comprising: the peptide consisting of the amino acid sequence of SEQ ID NO. 1; and glycyrrhizin.

4. The conjugate of claim 3, wherein the peptide and the glycyrrhizin are linked through a cross-linking agent.

5. The conjugate of claim 4, wherein the cross-linking agent is one or more selected from the group consisting of polyethylene glycol (PEG), 1,4-butanediol diglycidyl ether (BDDE), 1,3-butadiene diepoxide, divinyl sulfone (DVS), glycol chitosan, gelatin methacrylate, poly(lactic-co-glycolic acid) (PLGA), hyaluronic acid, and alginate.

6. The conjugate of claim 4, wherein the conjugate is bonded by a first bond by which the peptide and the cross-linking agent are bonded; and
a second bond by which the glycyrrhizin and the cross-linking agent are bonded.

7. The conjugate of claim 6, wherein the first bond is a disulfide bond.

8. The conjugate of claim 6, wherein the second bond is an amide bond.

9. A pharmaceutical composition for use in a method for preventing or treating obesity, the pharmaceutical composition comprising the conjugate of claim 3.

10. The composition for use according to claim 9, wherein the concentration of the composition is 10 µM to 500 µM.

11. The composition for use according to claim 9, wherein the composition inhibits hypertrophy of an adipocyte.

12. The composition for use according to claim 9, wherein the composition reduces the secretion amount of tumor necrosis factor-α (TNF-α).

13. The composition for use according to claim 9, wherein the composition increases the cell membrane expression level of ATP-binding cassette 1 transporter (ABCA1).

14. The composition for use according to claim 9, wherein the composition has a blood residual concentration that is1.5 to 3 times that of the control group.

15. A health functional food for use in a method for preventing or ameliorating obesity, the health functional food comprising the conjugate of claim 3.
